# EUROPEAN PATENT APPLICATION

(11) **EP 4 455 307 A1**
(43) Date of publication of application: **30.10.2024**
(21) Application number: 23170228.3
(22) Date of filing: 27.04.2023
(51) Int. Cl.: C12Q 1/6869

(54) **EX-SITU SEQUENCING OF RCA PRODUCT GENERATED IN-SITU**

(71) Applicant: Miltenyi Biotec B.V. & Co. KG, 51429 Bergisch Gladbach (DE)
(72) Inventor: HOSONO, Seiyu, 51429 Bergisch Gladbach (DE); ADEDIRAN, Jimmy, 51429 Bergisch Gladbach (DE); PINARD, Robert, 51429 Bergisch Gladbach (DE)
(74) Representative: Kisters, Michael Marcus

(57) **Abstract**

The invention is directed to a method for obtaining the sequence information of a target sequence from a tissue comprising at least one RNA or c-DNA strand comprising the steps
a. providing at least one first oligonucleotide comprising 50 - 1000 nucleic acids having a 5' and a 3' end
b. hybridizing the first oligonucleotide with its 5' and 3' ends to complementary parts of the at least one RNA or c-DNA strand
c. combining the 3' and 5' end of the hybridized first oligonucleotide with each other thereby obtaining a first single strand circular template
d. multiplying the first single strand circular template by a polymerase capable of rolling circle amplification into a plurality of concatemers thereby obtaining primary rolonies.
e. removing the primary rolonies from the sample
f.fragmenting the primary rolonies into a plurality of second oligonucleotides and hybridizing a first PCR primer and a second PCR primer at the 3 and 5' ends of the second oligonucleotides thereby obtaining third oligonucleotides
g. multiplying the third oligonucleotides by a polymerase capable of polymer chain reaction (PCR)
h. ligating the first PCR primer to the second PCR primer of the multiplied third oligonucleotides thereby obtaining second single strand circular templates
i.multiplying the second single strand circular templates by a polymerase capable of rolling circle amplification into a plurality of concatemers thereby obtaining secondary rolonies
j.determining the sequence of the secondary rolonies thereby obtaining the sequence information of the target sequence.

## Description

The present invention is directed to retrieving, extracting, and sequencing of a Rolling Circle Amplified (RCA) product generated on a tissue section from circular or padlock probes which hybridized to targeted regions of in-situ expressed mRNA and ligated with or without reverse transcribed targeted region of interest which may include a nucleotide change/variant or any other sequence of interest.

### BACKGROUND

Padlock oligonucleotides have proven to be very successful in polymerizing short portion of nucleic acids to which it has been hybridized to. Most padlock approaches begin by reverse transcribing the target into cDNA.

Padlock methods are for example disclosed in "Highly multiplexed subcellular RNA sequencing in situ" by Lee et al., Science. 2014 March 21; 343(6177): 1360-1363. doi:10.1126/science.1250212 or "Efficient In Situ Detection of mRNAs using the Chlorella virus DNA ligase for Padlock Probe Ligation" by Nils Schneider and Matthias Meier; February 5, 2020 -Cold Spring Harbor Laboratory Press.

A comprehensive assay for targeted multiplex amplification of human DNA sequences is published by Sujatha Krishnakumar et al.; PNAS sent for review February 19, 2008.

Further, WO2017143155A2 discloses multiplex alteration of cells using a pooled nucleic acid library and analysis thereof and WO2018045181A1 discloses Methods of generating libraries of nucleic acid sequences for detection via fluorescent in situ sequencing.

The published Padlock methods allow sequencing of DNA or RNA, but only in situ and do not allow for full target regions to be sequenced Recently in situ genome sequencing (IGS) has been described as a method to simultaneously sequence and image genomes within a sample. This method describes a workflow to localize unique molecular identifiers (UMIs) by short read in situ sequencing followed by amplicon dissociation, PCR and ex situ sequencing of amplicons associated to genomic sequences with UMIs by paired-end sequencing published by A. C. Payne et al., Science 10.1126/science.aay3446 (2020).

Microscopy imaging that allow for multiple mRNAs to be resolved at a single cell level provides valuable information regarding transcript amount and localization, which is a crucial factor for understanding tissue heterogeneity, the molecular development and treatment of diseases. Further, being able to identify potential mutations from mRNA for which the spatial information is know is also extremely valuable.

A method to obtain spatial information and sequencing for RNA or c-DNA is disclosed in EP 3936623. In this method, an oligonucleotide is hybridized to RNA or c-DNA to form a circular template, wherein the oligonucleotide (and later the circular template) comprises at least one region having a known sequence which is recognized by detection probes having the appropriate complementary sequence. By detection of the detection probe, special information of RNA or c-DNA on tissue can be obtained. In a variant of this method, the circular template can be fragmented and re-circularized to obtain second circular templates for further amplification. However, since this method is intended to obtain spatial information, the first and second circularization/amplification steps are performed on tissue.

### OBJECT OF THE INVENTION

The present invention is directed to a method for retrieving the Rolling Circle amplified (RCA) product generated on a tissue which carries the desired target nucleotide (genomic DNA or mRNA) and optionally a barcode or a unique molecular identifier which may serve as a spatial identifier.

This is accomplished by the use of a circle or a padlock molecule which is used to detect and hybridize to a desired target nucleotide (genomic DNA or mRNA) of interest. The desired sequence information is captured by a circle or a padlock molecule used to detect and hybridize to a desired target nucleotide (genomic DNA or mRNA) of interest on tissue. These circle or padlock molecule carries the desired target nucleotide (genomic DNA or mRNA) or the barcode or a unique molecular identifier serving as a spatial identifier. These circles or padlocks are RCA amplified directly on a tissue. The RCA product is then physically retrieved and extracted from the tissue, fragmented and the regions of interest are amplified by PCR of followed by a second round of circularization and RCA amplification. The RCA product is then sequenced using the NGS sequencing platform

By NGS sequencing the targeted region of interest on genomic DNA or mRNA, mutation or nucleotide variant can be analyzed. A spatial identifier, are also assigned to the location of the sequence of interest on the tissue

### SUMMARY

Accordingly, it was an object of the invention to provide a method to obtain sequence information of a target sequence from a tissue sample with a higher resolution than the known technologies.

Object of the invention is a method for obtaining the sequence information of a target sequence from a tissue comprising at least one RNA or c-DNA strand comprising the steps
a. providing at least one first oligonucleotide comprising 50 - 1000 nucleic acids having a 5' and a 3' end
b. hybridizing the first oligonucleotide with its 5' and 3' ends to complementary parts of the at least one RNA or c-DNA strand
c. combining the 3' and 5' end of the hybridized first oligonucleotide with each other thereby obtaining a first single strand circular template
d. multiplying the first single strand circular template by a polymerase capable of rolling circle amplification into a plurality of concatemers thereby obtaining primary rolonies.
e. removing the primary rolonies from the sample
f.fragmenting the primary rolonies into a plurality of second oligonucleotides and hybridizing a first PCR primer and a second PCR primer at the 3 and 5' ends of the second oligonucleotides thereby obtaining third oligonucleotides
g. multiplying the third oligonucleotides by a polymerase capable of polymer chain reaction (PCR)
h. ligating the first PCR primer to the second PCR primer of the multiplied third oligonucleotides thereby obtaining second single strand circular templates
i.multiplying the second single strand circular templates by a polymerase capable of rolling circle amplification into a plurality of concatemers thereby obtaining secondary rolonies
j.determining the sequence of the secondary rolonies thereby obtaining the sequence information of the target sequence.

The method of the invention is especially useful for quality control of sequencing methods. Accordingly, further objects of the invention are method for using the sequence information of the target sequence obtained to quantify a gene expression profile or a method for using the sequence information of the target sequence obtained to confirm the efficacy of a hybridization oligonucleotide and the target sequence selection.

Here we describe a method to (1) retrieve RCA product from a tissue section and (2) targeted PCR amplification of the RCA product which contains the region of interest with a nucleotide change/variant and/or barcode/unique molecular identifier.

The method of the invention is in part performed directly on tissue and in part after removal of the molecules containing the target sequence from the tissue. The "on tissue" steps comprise .
1. Retrieval and extraction of DNA of the RCA product from a tissue section
2. Targeted PCR amplification of the RCA product
3. Circularization of PCR product and RCA amplification
4. NGS Sequencing

### BRIEF DESCRIPTION OF THE DRAWINGS

Figure 1 shows the design of circular or padlock probe used in this method. The circle/padlock may contain a barcode or UMI identifier. The circle/padlock is hybridized to a tissue section which expressed the mRNA or genomic DNA of interest. Once hybridized, the padlock is ligation with SplintR Ligase to generate a circle. The circle can then be used to perform rolling circle amplification (RCA) to generate a detectable RCA product on the tissue.
Figure 2 shows the strategy to PCR amplify the region of interest of the RCA produced from a circle/padlock. The PCR primers with P1 and P2 adaptors hybridize to the flanking regions of the region of interest so that it can be amplified and sequenced.
Figure 3a and 3b shows a successful ex-situ sequencing of 4 gene transcripts from padlocks extracted from tissue. Every one of the padlocks, designed to detect the transcript of interest, was unambiguously identified by sequencing the targeted region of interest.

### DETAILED DESCRIPTION

Ex-situ sequencing of the RCA performed directly on tissue consists of a six step process. (1) RCA generation on tissue. (2) Retrieval of RCA rolonies and extraction of DNA from rolonies from a tissue section. (3) Targeted PCR amplification of the region of interest. The region of interest can be either a Barcode/UMI or a nucleotide change/variant in the target sequence. (4) Circularization of the PCR product. (5) RCA or the circle. (6) NGS Sequencing.

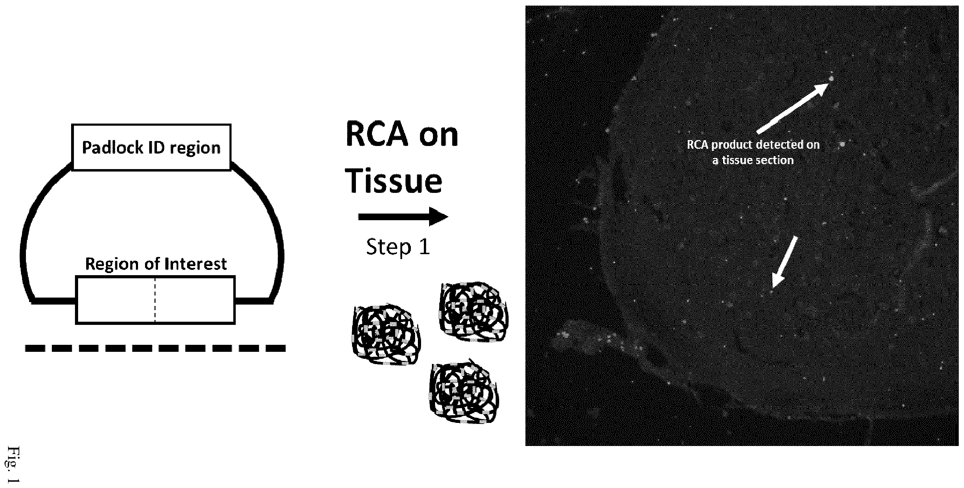

In Figure 1, two types of padlocks which hybridizes to an mRNA (dotted line) are depicted with a specific region of interest may be used. The region of interest can contain either a nucleotide position in the region which hybridizes to the mRNA with base mutation or variant, or a barcode of UMI in the padlock backbone region. The circle generated followed by ligation can serve as a substrate to generate RCA rolonies directly on tissue (Step 1 in Figure 1).

In a first embodiment, the 5' and the 3' ends of the first oligonucleotides are hybridized adjacent to complementary parts of the at least one RNA or c-DNA strand thereby obtaining the first single strand circular templates by direct ligation of the 5' and the 3' ends of the first oligonucleotides with each other.

In a second embodiment, the 5' and the 3' ends of the first oligonucleotides are hybridized to complementary parts of the at least one RNA or c-DNA strand with a gap of 2 to 100 nucleotides between the 5' and the 3' ends of the first oligonucleotides and obtaining the first single strand circular templates by filling the gap with nucleotides complementary to the RNA or c-DNA strand.

Preferable, the first oligonucleotide comprises a fragmentation sequence allowing the primary rolonies to be fragmented by a restriction enzyme or chemically.

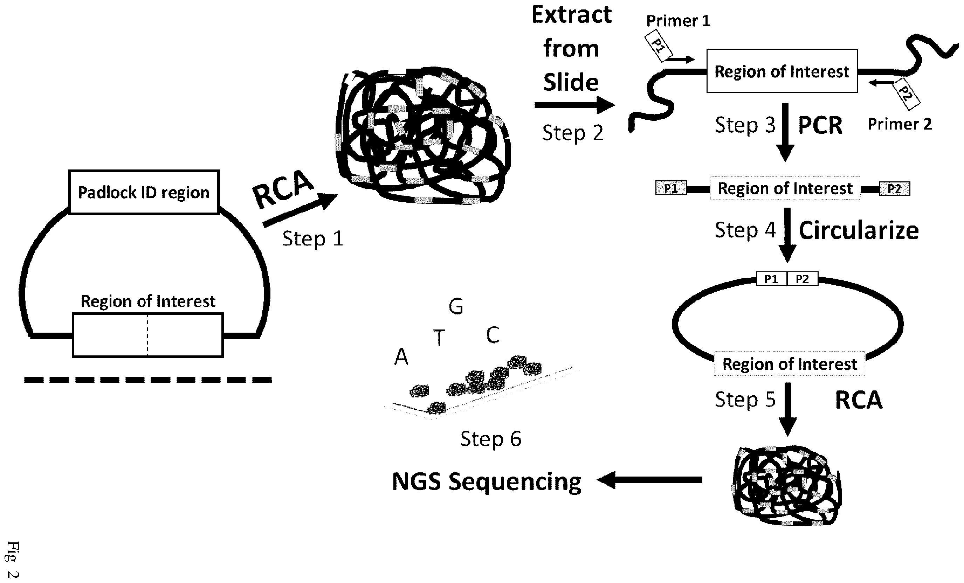

Extraction of DNA from RCA performed method to retrieve RCA product from a tissue section is described (Step 1 and 2 in Figure 2). First, tissue digestion is performed on the tissue section containing the RCA product by heating the sample in the presence of lysis buffer and Proteinase K.

The sample may be removed from the heat source and incubated with solid phase reversible immobilization (SPRI) beads and by using a magnet, the beads containing the nucleic acid can be easily washed.

Preferable, the eluant solution is added to the SPRI beads and after washing three times, a magnet is used to remove the SPRI bead and the supernatant is transferred to a tube. This tube contains the extracted RCA DNA eluant.

The extracted nucleic acid may be quantified using Nanodrop or Qubit.

Targeted PCR amplification is performed on the retrieved RCA product which contains the padlock junction region of interest with a nucleotide change/variant is described. Once the RCA DNA were extracted and quantified, the RCA DNA is used as a template for a PCR reaction using primer set (first PCR and second PCR primer) specific for the region flanking the region of interest (Step 3 in Figure 2).

For example, the sequence of the first PCR primer can be ACACGACGCTCTTCCGATCT**AAGGATACTCCGACGCGGCCGCA** and the second PCR primer can be GACGTGTGCTCTTCCGATCT**ACCCTTTACAAACACA**. The bold face type sequence hybridizes to the region flanking the region of interest is shown in Step 3 of Figure 2. P1 and P2 adapter portions are unique sequences which may be used for circularization in later step.

The PCR is performed for 25 cycles.

PCR product is purified using QiaQuick PCR product purification column, the DNA was quantified using Qubit assay.

The resulting PCR product with P1 and P2 adapters are circularized using a splint oligonucleotide which brings the two ends together (Step 4 in Figure 2). Preferable, the first PCR primer is ligated to the second PCR primer by providing splint DNA.

The circle is RCA amplified (Step 5 in Figure 2) and Rolonies are formed.

NGS sequencing can be performed with sequencing primer which binds to either P1 or P2 adaptor region (Step 5 in Figure 2).

NGS sequencing can determine either a mutation/nucleotide variant exist is the target region of interest, and by sequencing the padlock ID region, the location of the rolonies on the substrate can later on be correlated with the original position of the tissue. That means that the location of the gene on the tissue can be determined as well as the presence of a mutation or not via sequencing. In addition the gene expression profile can also be shown by the quantification of the sequencing read counts obtained.

Further, the spatial location of the first rolonies on the tissue is determined by imaging emission radiation of the at least one fluorescently labelled oligonucleotide bound to the first rolonies.

To this end, the first rolonies may be obtained by decorating (binding) the first rolonies with at least one fluorescently labelled oligonucleotide.

Further, the first oligonucleotide comprises a identification region comprised of a UMI sequence and or a barcode sequence to which the at least one fluorescently labelled oligonucleotide binds.

### EXAMPLES

Four mouse genes were study for which five different padlocks oligonucleotides probes were designed to be complementary to a portion of the corresponding mRNA transcripts. A total of 20 probes (four genes times 5 probes each) were hybridized against their respective targets in a mouse tissue section. The probes were provided in excess. The padlocks probes were then ligated enzymatically and RCA was performed directly on the tissue (in situ). The gene specific generated rolonies were detected by hybridizing fluorescently labelled oligonucleotides to the padlock identification region containing a UMI and or barcode sequences. As described in Step 1 above, the RCA rolonies were then extracted from the tissue and fragmented randomly. As described in Step 2, PCR reactions were performed using primers where one portion is complementary to the regions flanking the region of interest and the other portion contains generic sequences (P1 & P2). The resulting linear product with P1 and P2 adapters are circularized using a splint oligonucleotide which brings the two ends together. The circle is RCA amplified and secondary rolonies are formed.

The region of interest of RCA product are finally sequenced using an NGS Sequencer compatible with rolonies. Each sequence generated can be aligned and mapped to the four targeted gene transcripts.

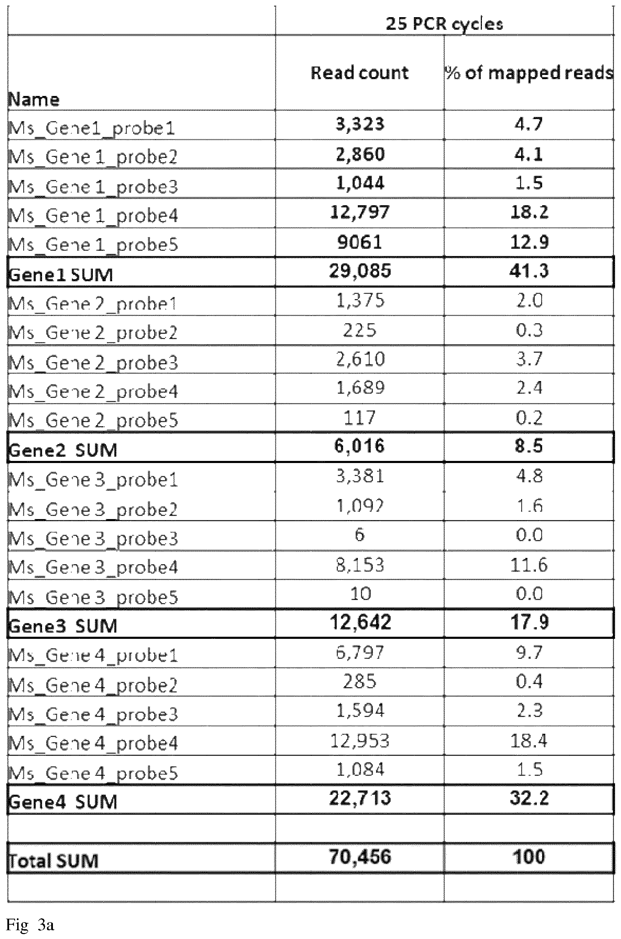

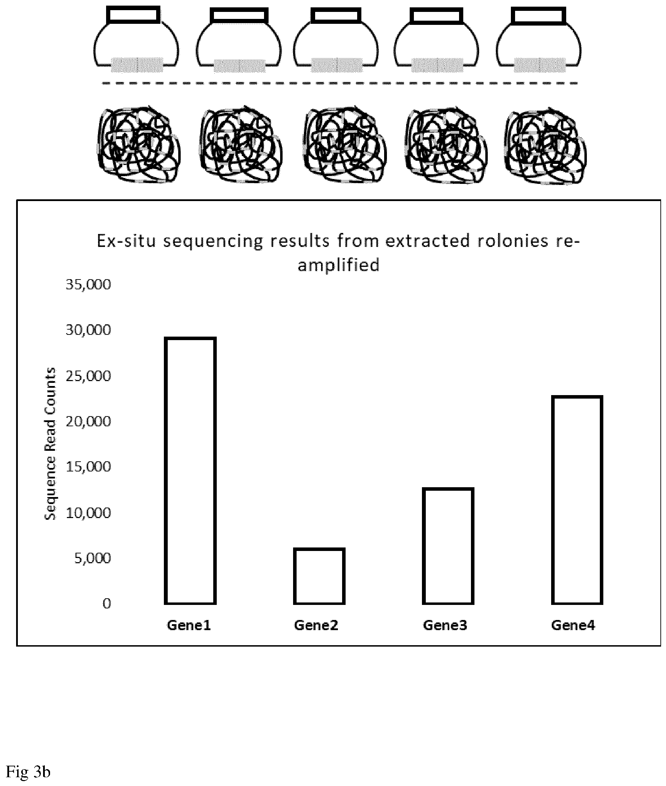

As shown in Figure 3 below, sequencing reads for all four genes were unambiguously detected with different read counts. These results shows that this method can be used as a tool to quantify the number of specific RCA products (rolonies) from the tissue that it was extracted from. In addition, and contrary to any other method, the efficacy of hybridization of the various padlock probes targeting the same transcript can be evaluated by quantifying the individual sequencing read counts of each individual probe. For example, for Gene 1, some probes are hybridizing more efficiently and therefore the design of the probes can be improved by looking at the sequencing read counts. It can also be used to quantify the gene expression profile as indicated in the graph of Fig 3 and confirm the hybridization approach performed using fluorescently labelled oligonucleotide on the primary rolonies generated on tissue

## Claims

1. A method for obtaining the sequence information of a target sequence from a tissue comprising at least one RNA or c-DNA strand comprising the steps
a. providing at least one first oligonucleotide comprising 50 - 1000 nucleic acids having a 5' and a 3' end
b. hybridizing the first oligonucleotide with its 5' and 3' ends to complementary parts of the at least one RNA or c-DNA strand
c. combining the 3' and 5' end of the hybridized first oligonucleotide with each other thereby obtaining a first single strand circular template
d. multiplying the first single strand circular template by a polymerase capable of rolling circle amplification into a plurality of concatemers thereby obtaining primary rolonies.
e. removing the primary rolonies from the sample
f. fragmenting the primary rolonies into a plurality of second oligonucleotides and hybridizing a first PCR primer and a second PCR primer at the 3 and 5' ends of the second oligonucleotides thereby obtaining third oligonucleotides
g. multiplying the third oligonucleotides by a polymerase capable of polymer chain reaction (PCR)
h. ligating the first PCR primer to the second PCR primer of the multiplied third oligonucleotides thereby obtaining second single strand circular templates
i.multiplying the second single strand circular templates by a polymerase capable of rolling circle amplification into a plurality of concatemers thereby obtaining secondary rolonies
j.determining the sequence of the secondary rolonies thereby obtaining the sequence information of the target sequence.

2. Method according to claim 1 **characterized in that** the 5' and the 3' ends of the first oligonucleotides are hybridized adjacent to complementary parts of the at least one RNA or c-DNA strand thereby obtaining the first single strand circular templates by direct ligation of the 5' and the 3' ends of the first oligonucleotides with each other.

3. Method according to claim 1 **characterized in that** the 5' and the 3' ends of the first oligonucleotides are hybridized to complementary parts of the at least one RNA or c-DNA strand with a gap of 2 to 100 nucleotides between the 5' and the 3' ends of the first oligonucleotides and obtaining the first single strand circular templates by filling the gap with nucleotides complementary to the RNA or c-DNA strand.

4. Method according to any of the claims 1 to 3 **characterized in that** the first PCR primer is ligated to the second PCR primer by providing splint DNA.

5. Method according to any of the claims 1 to 4 **characterized in that** the rolling circle amplifications (RCA) are activated by light and/or heat.

6. Method according to any of the claims 1 to 5 **characterized in that** the first oligonucleotide comprises a fragmentation sequence allowing the primary rolonies to be fragmented by a restriction enzyme or chemically.

7. Method according to any of the claims 1 to 6 **characterized in that** the first rolonies are decorated with at least one fluorescently labelled oligonucleotide.

8. Method according to claim 7 **characterized in that** the spatial location of the first rolonies on the tissue is determined by imaging emission radiation of the at least one fluorescently labelled oligonucleotide bound to the first rolonies.

9. Method according to claim 7 or 8 **characterized in that** the first oligonucleotide comprises a identification region comprised of a UMI sequence and or a barcode sequence to which the at least one fluorescently labelled oligonucleotide binds.

10. Method according to any of the claims 1 to 9 **characterized in that** the sequence information of the target sequence is used to quantify a gene expression profile.

11. Method according to any of the claims 1 to 10 **characterized in that** the sequence information of the target sequence is used to confirm the efficacy of a hybridization oligonucleotide and the target sequence selection.
